(19) Europäisches Patentamt
European Patent Office
Office européen des brevets

(11) **EP 4 008 247 A1**

(12) **EUROPEAN PATENT APPLICATION**

(43) Date of publication:
**08.06.2022 Bulletin 2022/23**

(21) Application number: **20211050.8**

(22) Date of filing: **01.12.2020**

(51) International Patent Classification (IPC):
**A61B 5/05** *(2021.01)* **A61B 5/00** *(2006.01)*

(52) Cooperative Patent Classification (CPC):
**A61B 5/05; A61B 5/7225**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **STEUNEBRINK, Tim Patrick**
**5656 AE Eindhoven (NL)**
• **DOODEMAN, Gerardus Johannes Nicolaas**
**5656 AE Eindhoven (NL)**
• **PEETERS, Wouter Herman**
**5656 AE Eindhoven (NL)**

(74) Representative: **Philips Intellectual Property & Standards**
**High Tech Campus 52**
**5656 AG Eindhoven (NL)**

(54) **INDUCTIVE SENSING SYSTEM FOR SENSING ELECTROMAGNETIC SIGNALS FROM A BODY**

(57) A physiological parameter inductive sensing system has a loop resonator which inductively couples with electromagnetic signals emitted from the body. The loop resonator forms part of an oscillator circuit, and negative feedback control is used to control the oscillator circuit, based on a measured oscillation amplitude. Within the feedback control loop, an analog to digital converter is used with a first number of bits (or trits), and successive outputs of the analog to digital converter are combined to derive an output value with a resolution of a second number of bits, greater than the first number of bits (or trits). The feedback control of the amplitude of the oscillator circuit is achieved using the output value.

FIG. 3

**Description**

FIELD OF THE INVENTION

This invention relates to an inductive sensing system for sensing electromagnetic signals from a body, and in particular a sensor for inductively coupling with a body for sensing electromagnetic signals.

BACKGROUND OF THE INVENTION

Sensors that measure mechanical activity are called kymographic sensors. Examples of kymographic biometric sensors are accelerometer-based biosensors, transthoracic impedance biosensors, radar-based biosensors, and photoplethysmography sensors.

Inductive sensors have the potential to become a game-changer in the field of patient monitoring, because the biometric signals are relatively motion tolerant. Inductive sensing has application in wearable patient monitors, motion tolerant respiration measurements, contactless patient monitoring, and spot-check measurements. Inductive sensing thus generally provides a means of non-invasive investigation of properties of a body.

In one advantageous area of application, inductive sensing can be used as a means of non-invasively investigating physiological characteristics, in particular heart and lung dynamics. Inductive sensing is based on magnetic induction and has several advantages over conductive and capacitive sensing.

An advantage compared to conductive sensing, such as bio-impedance measurements, is that adhesive electrodes are not required; sensing may be performed without contact and/or through non-conductive material, such as textile and plastic.

An advantage compared with capacitive sensing is that inductive sensing is based on magnetic fields rather than electric fields and as a result is more sensitive to changes at greater penetration depth inside the body, as opposed to those just occurring at skin level. This is because magnetic fields penetrate deeper into a body than electrical fields, and thus magnetic fields can be used to measure changes in properties deeper inside the body, whereas electrical fields are predominantly useful only for measuring effects at the surface of the skin such as changes in skin properties (e.g. permittivity) or movement of the skin (skin proximity).

Coil-based inductive sensors function by inductively coupling with electromagnetic signals (i.e. electromagnetic waves or oscillations), wherein propagation of the signals through the coil leads to a change in the current through the coil, which can be measured and used to sense properties of the propagated signal (including e.g. frequency spectrum, amplitude and phase pattern).

An electromagnetic excitation signal can be propagated into a body to be investigated. The excitation electromagnetic signal causes magnetic induction in the body, i. e. the generation of eddy currents in the tissue of the body due to the application of an external magnetic field. These eddy currents then in turn generate electromagnetic signals propagated out of the body which interact with the applied fields in a way that allows them to be sensed by the coil.

Movements of tissue in the body can manifest in changes in volumes of local regions of the tissue and in changes of the conductive or dielectric properties of a tissue. These changes then cause amplitude and/or phase modulations of the electromagnetic signal which is emitted out of the body in response to the electromagnetic stimulation. By monitoring these changes, movement and size change of elements within the body can be detected and tracked, and changes in the conductivity and dielectric properties can be tracked. For example, heart contractions manifest themselves mainly as movement of blood, and breathing mainly manifests itself as changes in the conductivity of the lung.

Recent inventions in the field of inductive sensing have enabled simple contactless measurements of the heart's and lung's mechanical activity, or the mechanical activity of a blood vessel like the radial artery in a human arm.

A first challenge of inductive sensing technology is however to comply with electromagnetic compatibility (EMC) regulations. Especially in a clinical setting, the restrictions are stringent. Since it is an active measurement in the radio frequency (RF) range, there will always be exposed electromagnetic fields (near field) and emitted electromagnetic radiation (far field). The electric RF current in the antenna loop is the dominant source of exposed and emitted electromagnetic fields.

A second challenge is to minimize the power consumption for wearable body sensors, because of limited battery capacity. Meanwhile, the signal-to-noise ratio is typically decreased when using lower power consumption circuitry, and care needs to be taken to enable a good enough signal quality for reliable measurement of vital signs.

There is a need to addresses these two challenges.

SUMMARY OF THE INVENTION

The invention is defined by the claims.

According to examples in accordance with an aspect of the invention, there is provided a physiological parameter inductive sensing system for sensing electromagnetic signals emitted from a body in response to electromagnetic excitation signals propagated into said body, the system comprising:

a loop resonator for inductively coupling with said electromagnetic signals emitted from the body;
an oscillator circuit which includes the loop resonator, for exciting the resonator to generate the electromagnetic excitation signals for propagating into said body;
an amplitude measurement circuit for measuring an amplitude of the emitted electromagnetic signals;
an analog to digital converter for digitizing the measured amplitude and generating a digital signal with a first number of bits or trits;
a counter for combining successive outputs of the analog to digital converter to derive an output value with a resolution of a second number of bits or trits, greater than the first number of bits or trits; and
a feedback controller for controlling the amplitude of the oscillator circuit based on the output value.

The sensing system uses an analog to digital converter, ADC, with a lower resolution than the resolution of the output value, which is the output measurement signal from the circuit. This is made possible by providing a counter (e.g. a digital integrator) inside a negative feedback loop which controls the amplitude of the oscillator. Thus, the amplitude is controlled, but the desired information relating to the amplitude of the oscillation (which conveys the information about the electromagnetic signals emitted from the body) is captured by the feedback control signal.

This feedback control signal stabilizes at a particular control level of the oscillator circuit, and this control level corresponds to a particular signal level emitted from the body. Thus, the feedback control signal functions as the measurement of the amplitude of the signal emitted from the body.

The first number of bits may be 1. In this way, a simple 1 bit quantizer may be used as the analog to digital converter, which compares the input with a threshold to determine a 0 or 1 output.

The first number of bits could also be 2, encoded in such a way that the ADC becomes a tri-state ADC. For example "00" means 'below reference', "01" and "10" means 'equal to reference', and "11" means 'above reference'.

The ADC could instead be a 1-trit ADC, which means it uses base-3 as a unit of information. This is equivalent to a 2 bit version that encodes a tri-state ADC.

The amplitude measurement circuit may comprise a circuit for measuring the imaginary part of the complex reflected inductance.

The feedback controller for example comprises a digital to analog converter. Thus, the feedback control loop uses analog control.

The feedback controller for example comprises a circuit for controlling a bias current within the oscillator circuit.

In one example, the circuit for controlling a bias current comprises a current mirror circuit for injecting a current into the oscillator circuit.

In another example, the oscillator circuit comprises a drive transistor and the circuit for controlling a bias current comprises a circuit for introducing losses to the driver transistor.

Thus, there are various ways to implement bias current control.

The loop antenna may comprise a loop capacitor, for setting the natural resonance frequency of the oscillator.

A frequency of the electromagnetic excitation signals is for example from 30 MHz to 1000 MHz. Maintaining a frequency below 1000 MHz optimizes penetration depth. At frequencies above 1000 MHz, penetration depth of electromagnetic signals start to become prohibitively small for measuring physiological parameters, e.g. lung or heart signals. Signal strength however becomes larger above a 30 MHz frequency.

A signal processing means may be provided for processing the output values over time to derive one or more physiological parameters. For example, the physiological parameters may comprise a heart rate and/or a breathing rate.

The invention also provides a method for sensing electromagnetic signals emitted from a body in response to electromagnetic excitation signals propagated into said body, the method comprising:

exciting a loop resonator to generate the electromagnetic excitation signals for propagating into said body, by controlling an oscillator circuit which includes the loop resonator;
measuring an amplitude of electromagnetic signals emitted from the body in response to the electromagnetic excitation signals;
converting the measured amplitude into a digital signal with a first number of bits or trits using an analog to digital converter;
combining successive outputs of the analog to digital converter to derive an output value with a resolution of a second number of bits, greater than the first number of bits or trits; and
controlling the amplitude of the oscillator circuit based on the output value.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows stimulation of a thorax of a subject in the vicinity of a resonator consisting of a loop antenna and a capacitor;
Figure 2 shows a conventional amplitude measuring circuit;
Figure 3 shows an amplitude measuring circuit of the invention;
Figure 4 shows an example of the amplitude measuring circuit; and
Figure 5 shows an example of the feedback control of the oscillator.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a physiological parameter inductive sensing system. A loop resonator inductively couples with electromagnetic signals emitted from the body. The loop resonator forms part of an oscillator circuit, and negative feedback control is used to control the oscillator circuit, based on a measured oscillation amplitude. Within the feedback control loop, an analog to digital converter is used with a first number of bits (or trits), and successive outputs of the analog to digital converter are combined to derive an output value with a resolution of a second number of bits, greater than the first number of bits (or trits). The feedback control of the amplitude of the oscillator circuit is achieved using the output value.

Before describing the invention, a summary of the principles behind inductive sensing will first be discussed. Further details can be found in WO 2018/127482.

An inductive coupling system makes use of a coil or wire which has induced across it a potential difference due to exposure to a time varying magnetic field. This principle may be used to measure the strength of electromagnetic signals generated within regions of a body by sensing changes in the inductance of a coil placed in proximity to the body, where these changes are detected based on changing resonance characteristics of the coil circuit.

Any electrical conductor exhibits a property of self-inductance. Self-inductance is the property of an electrical conductor by which a change in the current being driven through the conductor results in induction of an electromotive force in the conductor. According to Lenz' law, the induced electromotive force is in a direction such as to resist the change in current which is inducing it. It is hence commonly termed 'back-EMF'. Self-inductance can be understood as arising due to a magnetic flux induced as a result of the change in current (Ampere's law). This flux then interacts with the conductor itself to induce a back-EMF (Faraday's law of induction and Lenz' law).

The relationship between self-inductance L of a circuit, the voltage, v(t), and the current I(t) can be expressed as:

$$v(t) = L \frac{dI(t)}{dt} \qquad (1)$$

By using Faraday's law of induction to express v(t) as dΦB/dt (where ΦB is magnetic flux) and integrating with respect to time (assuming L to be time-constant), free-space self-inductance L for a coil of N turns can be expressed as:

$$L = N\Phi_B \, / \, I \qquad (2)$$

A resonator, for example comprising a single turn loop antenna, may be used to stimulate or excite a body with electromagnetic signals (waves) and to sense signals emitted back from the body in response to those excitation signals. The coil may be driven with an alternating current to generate the excitation signals for propagation into the body.

When the coil is brought into proximity with a body, the inductance L acquires an additional reflected inductance component, $L_r$, arising due to eddy currents induced in the stimulated body as a result of application of the excitation signals.

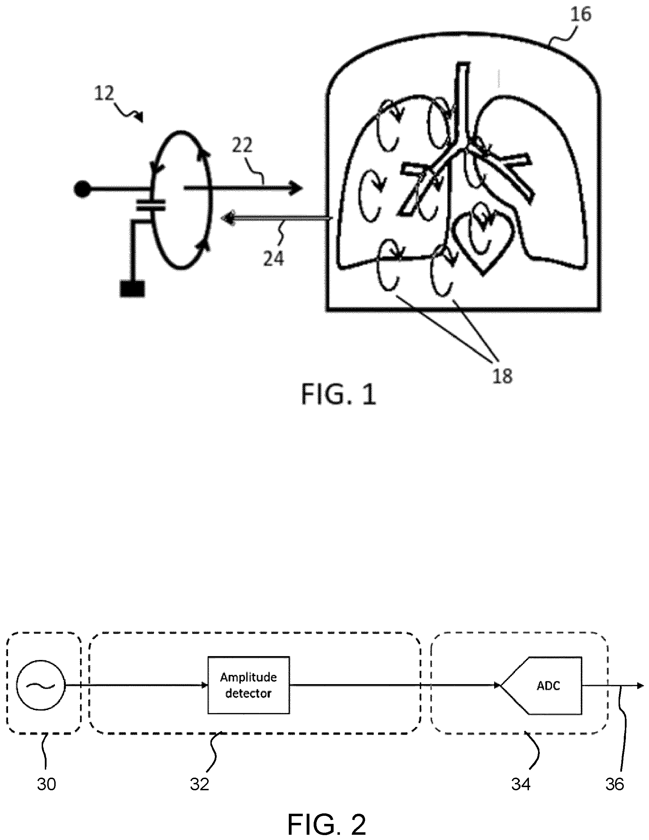

Figure 1 illustrates the operation of an inductive sensing system, which shows by way of example a loop antenna 12 being driven with an alternating current in proximity to a thorax 16 of a subject, so as to propagate electromagnetic signals 22 into the thorax. The loop antenna functions as a loop resonator. It may include a series capacitor used to tune the natural resonance frequency.

As a consequence, eddy currents 18 are induced within in the thorax. The eddy currents naturally arise due to Faraday's law of induction, whereby an electromotive force (EMF) is induced in a conducting medium in response to presence of a time-varying magnetic field.

These eddy currents in turn effectively make a contribution to the inductance of the loop antenna 12. This is because they themselves result in generation of a time-varying magnetic flux 24 of equivalent frequency to that generated by the primary antenna 12. These eddy-current fluxes combine with the primary flux of the antenna, resulting in a greater induced back-EMF in the antenna, and hence a larger measurable effective inductance.

The added component of inductance arising from the eddy currents is referred to as 'reflected inductance', $L_r$. The total inductance $L_t$ of the coil antenna 12 may be expressed as:

$$L_t = L_0 + L_r \tag{3}$$

where $L_0$ is the self-inductance of the coil antenna 12 and $L_r$ is the reflected inductance.

Reflected inductance can be defined as:

$$L_r \equiv \frac{1}{I} \oint_{\text{all turns}} \boldsymbol{A}_r \cdot d\boldsymbol{l} \tag{4}$$

where $\mathbf{A_r}$ is the reflected part of the electromagnetic vector potential (i.e. the part that is generated by the eddy currents 18 in the stimulated medium), and $I$ is the coil current. The reflected inductance is closely related to the reflected impedance $Z_r$. The relationship is $L_r = Z_r / i\omega$, where $\omega$ is the radial frequency of the electromagnetic excitation signals 22 (the time-varying field applied to the body).

The above integral expression can be understood by applying the relationship:

$$\nabla \times \boldsymbol{A}_r = \boldsymbol{B}_r \tag{5}$$

where $\boldsymbol{B}_r$ is the 'reflected' magnetic field and then applying Stokes' theorem to re-express equation (4) as:

$$L_r \equiv \frac{N}{I} \iint_S \boldsymbol{B} \cdot d\boldsymbol{s} = \frac{N\Phi_B}{I} \tag{6}$$

where N = number of turns. It can be seen that this corresponds to the form of the simplified expression for inductance outlined in equation (2) above.

The magnitude of the reflected inductance component gives an indication of the strength of the 'reflected' electromagnetic signals emitted back from the body. Stronger signals give higher signal to noise ratio, which improves the quality and reliability of the sensed signals. By seeking to optimize the strength of Lr, the signal to noise ratio may thereby be maximized.

In general, the reflected inductance, $L_r$, is complex, and can be expressed as

$$L_r = L_r' + iL_r'' \tag{7}$$

where $L_r'$ is related to a reactive impedance of the coil antenna and $L_r''$ is related to resistive impedance of the coil.

The addition of the reflected component of inductance $L_r$ leads to a detuning of the characteristics of the coil. In particular, both the natural radial frequency of the coil antenna circuit and the damping factor of the coil antenna circuit change. By measuring this detuning of the coil characteristics, the magnitude of the reflected inductance $L_r$ can be

determined, and the reflected signals measured.

In particular, the detuning of the characteristics of the coil as result of the addition of the reflected inductance can be expressed as follows:

$$\omega_{0,t} = \omega_{0,0} \sqrt{\frac{L_0}{L_0 + L_r'}} \qquad (8)$$

$$\zeta_t = \zeta_0 \sqrt{\frac{L_0}{L_0 + L_r'}} + \frac{-L_r''}{2(L_0 + L_r')} \qquad (9)$$

where $\omega_{0,0} = \sqrt{\frac{1}{CL_0}}$ is the undamped natural radial frequency of the coil circuit in free space, $\omega_{0,t}$ is the natural undamped radial frequency of the coil circuit in the presence of a medium or body (the subscript $t$ standing for 'total'), $\zeta_0 = \frac{\frac{R_0}{\omega_{0,0}}}{2L_0}$ is the damping factor in free space, $\zeta_t$ is the (total) damping factor in the presence of a medium, $L_r'$ is the real part of the reflected inductance defined in equation (7) and $L_r''$ is the imaginary part of the reflected inductance defined in equation (7).

It can be seen that the detuned natural radial frequency depends only on the real part of the reflected inductance $L_r'$. The detuned damping factor depends also upon the imaginary part of the reflected inductance $L_r''$.

For simplicity, it is preferable to work with geometrically normalized quantities. Accordingly, a 'characteristic' self-inductance $\hat{L}_0$ and reflected inductance $\hat{L}_r$ can be defined as follows:

$$\hat{L}_0 \equiv \frac{L_0}{lN^2} \qquad (10)$$

$$\hat{L}_r \equiv \frac{L_r}{lN^2} \qquad (11)$$

where $l$ = circumference of a single turn of the coil, $N$ = number of coil windings, $L_0$ is free-space self-inductance (real), $L_r$ is reflected inductance (complex), and where $L_r$ is defined as in equation (4) above. The benefit of using the geometrically normalized quantities lies in the fact that $\hat{L}_0$ is independent of system size and the number of turns in the antenna coil.

Using these characteristic quantities, the detuning of the characteristics of the coil as a result of the addition of the reflected inductance can be expressed as follows:

$$\omega_{0,t} = \omega_{0,0} \sqrt{\frac{\hat{L}_0}{\hat{L}_0 + \hat{L}_r'}} \qquad (12)$$

$$\zeta_t = \zeta_0 \sqrt{\frac{\hat{L}_0}{\hat{L}_0 + \hat{L}_r'}} + \frac{-\hat{L}_r''}{2(\hat{L}_0 + \hat{L}_r')} \qquad (13)$$

where $\omega_{0,0} = \sqrt{\frac{1}{CL_0}}$ is the undamped natural radial frequency of the coil circuit in free space, $\omega_{0,t}$ is the natural undamped radial frequency of the coil circuit in the presence of a medium or body (the subscript $t$ standing for 'total'),

$$\zeta_0 = \frac{\frac{R_0}{\omega_{0,0}}}{2L_0}$$

is the damping factor in free space, $\zeta_t$ is the (total) damping factor in the presence of a medium, $\hat{L}'_r$ is the real part of the characteristic reflected inductance defined in equation (9) and $\hat{L}''_r$ is the imaginary part of the characteristic reflected inductance defined in equation (9).

Thus, it can be seen from the analysis above that since the reflected inductance is a complex quantity, it affects both the frequency and amplitude of the oscillator. Most inductive sensors focus only on the frequency, while in fact the amplitude is an independent signal that contains independent information. This amplitude variation is manifested in the damping factor described above, as the resonator damping modulates the oscillator amplitude. The amplitude variation is thus in particular expressed in the imaginary impedance component.

It is however more subtle to measure the amplitude accurately at low oscillator amplitudes (required for EMC compliance) and low power consumption.

Figure 2 shows the most straightforward way to measure amplitude, and hence the damping. The input signal source is represented as an oscillator 30. The swing of the signal (i.e. the oscillator tank swing) is measured by an amplitude detector 32 and digitized in an analog to digital converter 34 to generate the amplitude measurement output 36.

Figure 2 is an amplitude demodulation system. Such a system will have a large dynamic range (DR) to cover the damping measurement from free space to application of the sensor to the body. This requires both high linearity and high resolution/low noise electronics, consuming significant amounts of power. Most of this dynamic range is not used once the sensor is applied to the body, but for every digitization step, the full dynamic range is cycled to determine the digital output bit stream.

In addition to the high power consumption, the loop antenna 12 will radiate the maximum amount of power when it is in free space, making it harder to comply with EMC regulations and as well as decreasing the signal-to-noise ratio (SNR) of the measurement when applied to the body.

Thus, there two challenges, relating to electromagnetic compatibility (EMC) and power consumption. The invention is based on an understanding of the technical origins of these two challenges and provides a signal measurement solution which addresses both challenges.

In respect of EMC, the oscillator amplitude (and thereby the current in the loop) quickly exceeds the maximum current when it is not applied to a body, especially if the loop resistance is small. Also, when the loop is applied to the body, the total resistance of the loop increases which increases thermal noise and decreases the signal strength in free-running oscillators (i.e. oscillators without amplitude control).

In respect of power consumption, the electronics for the analog-to-digital converter (ADC) are power hungry, because to cover the full dynamic range, a multi-bit ADC is needed. The multibit ADC has many components that need to be powered simultaneously, and for every ADC cycle, the full dynamic range needs to be covered requiring many sub-cycles that are repeated every sample to be digitized. Also, to cover such a large dynamic range, the ADC needs to have great linearity over the full range, increasing the power consumption as well.

The invention is based on the use of an analog to digital converter with a reduced number of bits or trits, i.e. with a lower resolution than the resolution of the output value (i.e. the measurement signal provided by the circuit). What is meant by this is that the output of the analog to digital converter has a first number of bits (or trits). However, the output value of the overall circuit (the measurement signal) encodes the measured amplitude as an output value with a second, larger, number of bits. Thus the ADC has an output resolution that would be insufficient alone to define the measurement output. There is thus a lower resolution for the ADC than for the digital output and this is possible because the ADC output is integrated over time to improve accuracy and create a higher resolution measurement output. The ADC is only used to determine whether the signal is above or below (or at) the reference value.

One option is an analog to digital converter implemented as a 1-bit quantizer. A 1-bit quantizer is extremely low-power, but it has the disadvantage that it does not have the possibility to indicate that the exact right amplitude has been achieved. It always toggles between 'go up' and 'go down', which is a source of noise. This problem may be solved by using a 1-trit quantizer, which has an information base of 3, i.e., "go up", "don't do anything", and "go down". The result is then be cleaner feedback signal at a very minor additional cost compared to a 1 bit quantizer.

A similar effect can be achieved by using a 2-bit quantizer, whose semantics are encoded as "00"= go up, "01"="10"="don't do anything", and "11"="go down". This could be advantageous over a 1-trit quantizer because of readily available binary electronic semiconductor technologies.

Instead of open loop control as in Figure 2, a negative feedback loop is provided, with a counter (digital integrator) inside the negative feedback loop controlling the amplitude of the oscillator.

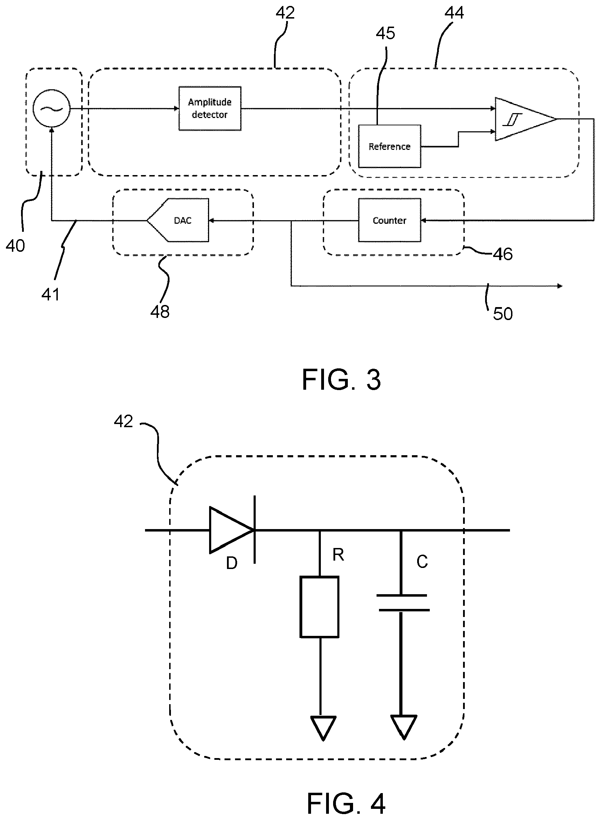

Figure 3 shows the amplitude detection circuit of the invention.

The signal source is again represented as an oscillator 40. In this case, the oscillator is not free running, but is an amplitude controlled oscillator, having an amplitude control input 41.

The detected signal is provided to a amplitude detector 42 again functioning as an amplitude demodulator. The amplitude detector 42 is used to measure the imaginary part of the complex reflected inductance.

Figure 4 shows an example of the amplitude detection circuit, implemented as a peak detector circuit. Measuring the imaginary part of the complex reflected inductance involves demodulating an amplitude modulated signal, i.e. the oscillator tank swing is modulated by the physiological signal from the body.

Amplitude demodulating circuits are well known and Figure 4 shows a most simple example in the form of a peak detector consisting of a diode D and a low-pass filter R,C. The RC values are chosen such that RF ripple from the carrier is minimized but the filter can react fast enough to changes in the physiological signal. The amplitude detector' block is thus implemented by an amplitude demodulator.

Returning to Figure 3, the measured amplitude signal is provided to a low-resolution ADC 44, for example a 1 bit quantizer. The quantizer compares its input signal to a reference 45 as part of the quantization process. This uses much less power than a full-resolution ADC, because it has fewer powered electronic sub-components.

To achieve the intended digital resolution of the output value, a counter 46 (a digital integrator) adds up the individual results of the quantizer.

The output of the counter 46 is the measurement output 50, and it is also applied to a digital to analog converter 48 so that an analog control signal is applied to the oscillator 40, thereby closing the negative feedback loop.

The digital to analog converter 48 is one part of a feedback controller. The analog signal 41 interacts with the oscillator circuit, and a part of the oscillator circuit thus also functions as part of the feedback controller. Options for implementing the feedback control are discussed below.

The negative feedback loop controls the amplitude to remain at a constant value, dependent on the reference 45 applied within the analog to digital converter 44. The analog to digital converter may, for example, comprise an array of switchable resistors.

Within the negative feedback loop, the ADC 44 when implemented as a 1-bit quantizer just determines whether the peak detector output is above or below the reference value. In order to obtain a peak detector output at the same level as the reference, the oscillator amplitude needs to be adjusted. The amount of adjustment is controlled by the counter (digital integrator) which will count up or down by one based on the ADC output until the reference value is reached. In case of a tri-state quantizer instead of a 1-bit quantizer, this results in the quantizer output to be 0. A 1-bit quantizer will keep toggling between 0 and 1 with the amplitude detector output around the reference level.

By using the counter output as the output value, the circuit avoids losing information contained in the amplitude measurement, for example as would be the case if the oscillator amplitude is limited but without a control loop.

EMC is achieved by using the integrated digital result of the amplitude measurement to control the amplitude of the oscillator and thereby limit the amplitude of the oscillator signal. This amplitude limiting function of the circuit addresses the issue of a high radiated power by the loop antenna and decreased signal to noise ratio (SNR) when the sensor is applied to the body. The oscillator amplitude is in this way kept at a constant value regardless of the amount of damping of the oscillator.

The measured amplitude itself thus no longer contains the information of the biometric signal being measured; this information has been transferred to the feedback control signal which thus defines the output value of the circuit (but still in the digital domain).

The ADC 44 may be a 1 bit quantizer as mentioned above. The ADC 44 may instead be a 2-bit quantizer, or a multi-bit quantizer. The ADC 44 may instead be a 1-trit quantizer or a multi-trit quantizer. However, the quantizer in all cases has a resolution lower than the output resolution, i.e. the number of levels of the output value 50 as explained above.

The ADC 44 determines (at least) whether the oscillator amplitude is above or below the reference level. Accordingly, the counter 46 will increase or decrease its value in order to increase or decrease the oscillator amplitude. The quantizer electronics is now much less complex and no longer requires a large dynamic range (only some dynamic range is needed around the reference level).

The measurement output is in this way derived from multiple operations of the quantizer and hence built up over time. Thus, the loop frequency of the circuit is higher than the highest frequency to be tracked of the amplitude being measured.

The loop frequency of the circuit depends on the resolution of the counter and hence digital output. A higher resolution means that the step of one count is smaller. In order to adjust the oscillator such that the amplitude detector is always at the reference level, faster adjustment is needed with smaller steps. If, for example, a triangular waveform is to be followed with a frequency of 1 Hz covering 100 LSB, adjustments will need to be made at 200 Hz (counting up 100 times and counting down 100 times). If the same signal is to be tracked with a resolution of 10 LSB, the loop frequency can be lower at 20 Hz.

If the input signal is not linear, but for example a sinusoidal signal, the minimum frequency of the loop is determined by the steepest part of the derivative of the input signal.

There could also be a tradeoff between the allowed error and loop frequency. If larger temporary errors are tolerated a slightly lower loop frequency may be used. For example, in the case of a sinusoidal signal it may not be possible to track the signal within 1 LSB when the derivative is high (at the zero crossings of the sine curve) but the tracking will catch up when the sine curve is near its peak value with a decreasing derivative.

Using a multi-bit quantizer as the ADC 44 enables determination of whether the amplitude is one or more LSBs from the reference, and in this way the counter can be adjusted with adjustments larger than 1, catching up more quickly than with a 1-bit quantizer.

Thus, the exact frequency of the loop depends on the resolution and bandwidth of the output signal, allowed error and the resolution of the quantizer. The loop frequency will always be higher than the output bandwidth. By way of example, the loop frequency is typically in the range of 1-10kHz for a realistic system.

For combined respiration and pulse measurements the bandwidth of the signal to be detected is typically 33 Hz.

This large frequency margin allows the required output resolution to be formed by an iterative integration process.

When there are changes in the body signal being measured, the quantizer delivers difference outputs successively until the feedback loop has adapted to the new input signal. By combining/counting these difference signals, the required change in the oscillator control signal 41 is recorded, and this corresponds to the measured amplitude.

The result of this architecture is that the RF emission in free space is reduced and the SNR when the sensor is applied to the body is increased. Also, not every bit is digitized for every digitization step, since the counter only increases or decreases the least-significant-bit (LSB) of the output value, resulting in a much more efficient digitization scheme.

The counter 46 only consumes power when it is switching (i.e. increasing or decreasing its output value) when implemented using CMOS logic, making this power efficient as well.

The amplitude of the oscillator 40 can be controlled in various ways.

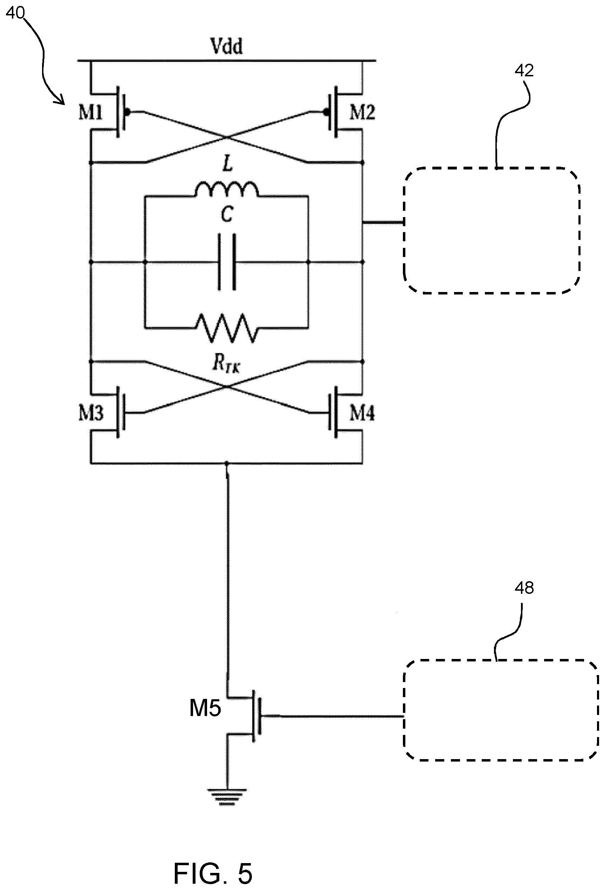

Figure 5 shows an example of the oscillator circuit 40 with the output provided to the amplitude detector 42 (of Figure 3) and the feedback signal received from the digital to analog converter 48 (of Figure 3).

The oscillator circuit 40 is a well known cross-coupled CMOS oscillator, comprising two parallel circuit branches with cross-coupled pull up transistors M1, M2 and cross-coupled pull down transistors M3, M4. The oscillator tank is a parallel LC circuit connected between the branches. There is also a resistance R which is the equivalent parallel resistance representing the losses of L and C. The resistance R is thus not normally the result of a physically present component in the circuit, but such a resistor may be used to introduce a deliberate switchable loss (as discussed below).

The tank voltage swing is measured using the amplitude detector 42, e.g. a peak detector as explained above.

A bias current drawn from the oscillator circuit is controlled by a tail transistor M5. The bias current is directly related to the tank voltage swing. The digital to analog converter 48 can be implemented in different ways to control the bias current. This could be, but is not limited to, a current mirror with adjustable current or a voltage DAC controlling the gate-source voltage of the tail transistor.

Instead of a tail transistor, an adjustable resistor may be used, controlled by the output of the counter.

In the example of Figure 5, the amplitude control is achieved by a voltage digital to analogue converter 48 controlling the gate-source voltage of the tail transistor M5.

However, there are other options for controlling the amplitude of the oscillator circuit. One example is to add a load to the antenna loop. As explained above, the loop impedance can be expressed in terms of inductance (L), capacitance (C) and resistance (R). Generally speaking, the series losses of the inductor increase when applied to the body, which means the equivalent parallel resistance decreases compared to when it is in free space, and it also varies with the physiological signal that is being measured.

The resistance R directly influences the tank voltage swing of the oscillator. Therefore an option is to control the amplitude by controlling the resistance of the loop. Additional resistors in series or parallel to the loop can be switched on or off in such a way that the total loop resistance is always the same. This would mean that for the highest imaginary reflected inductance value, no or little extra resistance is added, but in free space when the imaginary part of the reflected impedance is low, more resistance is added to end up with the same total resistance.

The amount of extra resistance needed is then a measure for the physiological signal that is being measured. Having a constant loop resistance keeps the tank voltage swing, and therefore the current through the loop, at a constant level, reducing the radiated magnetic field making it EMC compliant.

Another example is to introduce controllable losses to the source of one or more of the oscillator transistors.

The amplitude is typically controlled in an analog manner, hence the need for the digital to analog converter 48. The output connects to the oscillator tank for example to increase the damping in free space, or is connected to a current mirror to control the bias current of the oscillator, as mentioned above.

A frequency of the electromagnetic excitation signals is for example from 30 MHz to 1000 MHz. Other suitable ranges are 100 MHz to 1000 MHz, 30 MHz to 500 MHz and 100 MHz to 500 MHz.

The measurement output is typically displayed for an end-user which could be a clinician, nurse, or the patient him/herself, or a healthy consumer for example in sleep and sport applications.

The system may in particular be a vital sign sensing system. Vital signs may include for instance heart rate, pulse rate, breathing capacity, breathing rate, stroke volume, stroke volume variations, cardiac output, or aortic or arterial pulse height/pressure/diameter modulations. To derive these vital signs, a signal processing means may be provided for processing the output values overtime to derive the desired one or more physiological parameters.

The invention is of interest for patient monitoring applications, telemetry, and spot-check monitoring. It may be integrated into a wearable system, such as a chest patch or a wrist-worn device or it may be part of a mattress based monitoring system. The invention is for example of particular interest for neonatal monitoring, sleep monitoring, or obstetrical monitoring.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A physiological parameter inductive sensing system for sensing electromagnetic signals emitted from a body in response to electromagnetic excitation signals propagated into said body, the system comprising:

   a loop resonator (12) for inductively coupling with said electromagnetic signals emitted from the body;
   an oscillator circuit (40) which includes the loop resonator for exciting the resonator to generate the electromagnetic excitation signals for propagating into said body;
   an amplitude measurement circuit (42) for measuring an amplitude of the emitted electromagnetic signals;
   an analog to digital converter (44) for digitizing the measured amplitude and generating a digital signal with a first number of bits, or trits;
   a counter (46) for combining successive outputs of the analog to digital converter (44) to derive an output value (50) with a resolution of a second number of bits, greater than the first number of bits; and
   a feedback controller (48) for controlling the amplitude of the oscillator circuit based on the output value.

2. The system of claim 1, wherein digital signal generated by the analog to digital converter comprises a 1 bit signal.

3. The system of claim 1, wherein digital signal generated by the analog to digital converter comprises a 2 bit signal.

4. The system of claim 1, wherein digital signal generated by the analog to digital converter comprises a 1 trit signal.

5. The system of any one of claims 1 to 4, wherein the amplitude measurement circuit comprises a circuit for measuring the imaginary part of the complex reflected inductance.

6. The system of claim 5, wherein the amplitude measurement circuit comprises a peak detector circuit.

7. The system of any one of claims 1 to 6, wherein the feedback controller (48) comprises a digital to analog converter.

8. The system of any one of claims 1 to 7, wherein the feedback controller (48) comprises a circuit for controlling a bias current within the oscillator circuit (40).

9. The system of claim 8, wherein the circuit for controlling a bias current comprises a current mirror circuit for injecting a current into the oscillator circuit.

10. The system of claim 8, wherein the oscillator circuit comprises a drive transistor and the circuit for controlling a bias current comprises a circuit for introducing losses to the driver transistor.

**11.** The system of any one of claims 1 to 10, wherein the loop antenna comprises a loop capacitor.

**12.** The system of any one of claims 1 to 11, wherein a frequency of the electromagnetic excitation signals is from 30 MHz to 1000 MHz.

**13.** The system of any one of claims 1 to 12, comprising a signal processing means for processing the output values over time to derive one or more physiological parameters.

**14.** The system of claim 13, wherein the physiological parameters comprise a heart rate and/or a breathing rate.

**15.** A method of for sensing electromagnetic signals emitted from a body in response to electromagnetic excitation signals propagated into said body, the method comprising:

exciting a loop resonator (12) to generate the electromagnetic excitation signals for propagating into said body, by controlling an oscillator circuit (40) which includes the loop resonator;
measuring an amplitude of electromagnetic signals emitted from the body in response to the electromagnetic excitation signals;
converting the measured amplitude into a digital signal with a first number of bits or trits using an analog to digital converter (44);
combining successive outputs of the analog to digital converter (44) to derive an output value (50) with a resolution of a second number of bits, greater than the first number of bits or trits; and
controlling the amplitude of the oscillator circuit based on the output value.

FIG. 1

FIG. 2

FIG. 3

FIG. 4

FIG. 5

Europäisches
Patentamt

European
Patent Office

Office européen
des brevets

# EUROPEAN SEARCH REPORT

Application Number

EP 20 21 1050

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2018/127482 A1 (KONINKLIJKE PHILIPS NV [NL]) 12 July 2018 (2018-07-12)<br>* page 28, line 23 - page 30, line 25; figures 1,7 *<br>* the whole document *<br>----- | 1-15 | INV.<br>A61B5/05<br>A61B5/00 |
| A | US 2014/095102 A1 (POTYRAILO RADISLAV ALEXANDROVICH [US] ET AL) 3 April 2014 (2014-04-03)<br>* paragraphs [0115] - [0116]; figure 13 *<br>* paragraphs [0122] - [0126] *<br>----- | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 21 May 2021 | Furlan, Stéphane |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 21 1050

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2021

| Patent document cited in search report | Publication date | Patent family member(s) | Publication date |
|---|---|---|---|
| WO 2018127482 A1 | 12-07-2018 | CN 110167441 A<br>EP 3565465 A1<br>JP 2020505970 A<br>RU 2019125246 A<br>US 2019336014 A1<br>WO 2018127482 A1 | 23-08-2019<br>13-11-2019<br>27-02-2020<br>09-02-2021<br>07-11-2019<br>12-07-2018 |
| US 2014095102 A1 | 03-04-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018127482 A